# FASCICULE DE BREVET EUROPEEN

(11) **EP 0 629 413 B1**
(45) Date de publication et mention de la délivrance du brevet: **29.12.1997**
(21) Numéro de dépôt: 94420071.6
(22) Date de dépôt: 01.03.1994
(51) Int. Cl.: A61M 1/36, A61M 1/34

(54) **Dispositif et procédé de contrôle de la balance des fluides sur un circuit extracorporel de sang**
Vorrichtung und Verfahren zum Steuern der Flüssigkeitsbilanz in einem extracorporalen Blutkreislauf
Apparatus and process for the control of the fluid equilibrium in an extracorporeal blood circuit

(30) Priorité: 22.03.1993 FR 9303468
(43) Date de publication de la demande: 21.12.1994
(73) Titulaire: HOSPAL INDUSTRIE, F-69883 Meyzieu Cédex (FR)
(72) Inventeur: Bene, Bernard, F-69540 Irigny (FR); Goux, Nicolas, F-69290 Craponne (FR)

(56) Documents cités:
- EP-A- 0 115 835
- EP-A- 0 452 229
- EP-A- 0 490 212
- WO-A-79/01121
- WO-A-91/05576
- DE-A- 3 620 270
- US-A- 4 964 976

## Description

La présente invention est relative au domaine du traitement du sang en circulation extracorporelle et concerne notamment un appareil de traitement du sang selon la préambule de la revendication 1.

Un tel appareil est décrit dans la demande de brevet internationale WO 79/01121.

Plus particulièrement, l'invention a trait au traitement du sang donnant lieu à l'extraction d'une certaine quantité de liquide compensée au moins partiellement par la réinjection d'un liquide de substitution, ainsi que cela est par exemple le cas dans les domaines de l'hémofiltration ou de l'hémodiafiltration.

Selon l'art antérieur il est connu de contrôler l'égalité entre la quantité de liquide retirée du patient et celle injectée grâce à un système de peson. Pour cela, le liquide retiré est recueilli dans une poche alors que le liquide de substitution injecté l'est à partir d'une poche. Chacune des poches est accrochée à un peson. Le débit de réinjection du liquide de substitution est alors commandé en fonction des informations fournies par le peson. Un tel système. s'il est fiable, présente cependant l'inconvénient d'appartenir aux techniques coûteuses et difficiles à mettre en oeuvre.

Dans le cas où le débit de liquide retiré du patient est fixé grâce à une pompe et où l'injection du liquide de substitution est également commandée par une pompe, il est connu de contrôler l'équilibre entre la quantité de liquide retiré et la quantité de liquide injecté en fixant les débits fournis par les pompes. Ce contrôle débitmétrique présente l'avantage d'être plus simple à mettre en oeuvre et moins coûteux que le contrôle gravimétrique; il a cependant comme inconvénient de ne pas être parfaitement fiable, une dérive pouvant exister entre le débit apparent fourni par une pompe et son débit réel.

Le but de l'invention est donc de pallier ces inconvénients et de proposer un dispositif et un procédé de contrôle de l'équilibre des quantités de liquide injecté et retiré du sang, qui soit fiable, peu coûteux et simple à mettre en oeuvre.

Un autre but de l'invention est de proposer un dispositif et un procédé de contrôle qui puissent être mis en oeuvre durant la séance de traitement du sang ou lors des opérations de vérification de la machine par le service de maintenance.

Pour atteindre ces différents buts, la présente invention a pour objet un appareil de traitement du sang comprenant :
- un échangeur à membrane ayant un premier et un second compartiments séparés par une membrane;
- un circuit pour circulation extracorporelle de sang, comprenant:
   - une première canalisation connectée à une entrée du premier compartiment et destinée à être connectée à un patient;
   - une seconde canalisation connectée à une sortie du premier compartiment et destinée à être connectée au patient;
   - une canalisation de dérivation reliant la première canalisation à la seconde canalisation de façon à définir une boucle de recirculation;
- des moyens d'obturation pour obturer la canalisation de dérivation;
- des moyens d'injection pour injecter un liquide dans le circuit pour circulation extracorporelle de sang ;
- des moyens d'extraction pour provoquer l'ultrafiltration de liquide plasmatique dans le second compartiment au travers de la membrane ;
   caractérisé en ce que :
- les moyens d'injection sont reliés à la boucle de recirculation pour y injecter un liquide de substitution destiné à compenser au moins en partie le liquide ultrafiltré,
   et en ce qu'il comporte:
- des moyens d'obturation pour obturer la première canalisation et la seconde canalisation de façon à isoler le patient de la boucle de recirculation ;
- des moyens de mesure de pression connectés à la boucle de recirculation pour produire un signal indicatif de la différence entre un débit du liquide infusé et un débit de liquide ultrafiltré lorsque la boucle de recirculation est isolée du patient.

La présente invention a également pour objet un procédé pour régler l'injection d'un liquide et l'extraction d'un liquide dans et hors d'un circuit extracorporel de sang connecté à un patient, ce procédé comprenant des étapes de :
- isoler temporairement une boucle de recirculation par rapport au patient, la boucle comprenant une portion d'une première canalisation reliant le patient à un échangeur à membrane, l'échangeur à membrane, une portion d'une seconde canalisation reliant l'échangeur à membrane au patient, et une canalisation de dérivation connectant la première canalisation à la seconde canalisation;
- faire circuler le sang dans la boucle;
- mesurer la pression dans la boucle pendant un intervalle de temps, et, lorsque la pression mesurée varie pendant l'intervalle de temps,
- modifier soit le débit d'injection de liquide dans la boucle, soit le débit d'extraction de liquide hors de la boucle de façon que la pression dans la boucle soit sensiblement constante, ou évolue selon une évolution souhaitée.

De nombreux avantages de la présente invention apparaîtront à la lecture de la description qui va suivre en référence aux figures qui illustrent, de façon schématique et sans échelle déterminée, différents modes de réalisation de la présente invention.
La figure 1 représente un premier mode de réalisation de l'invention en relation avec la technique d'hémofiltration.
La figure 2 représente un deuxième moyen de réalisation de l'invention en relation avec la technique d'hémodiafiltration.

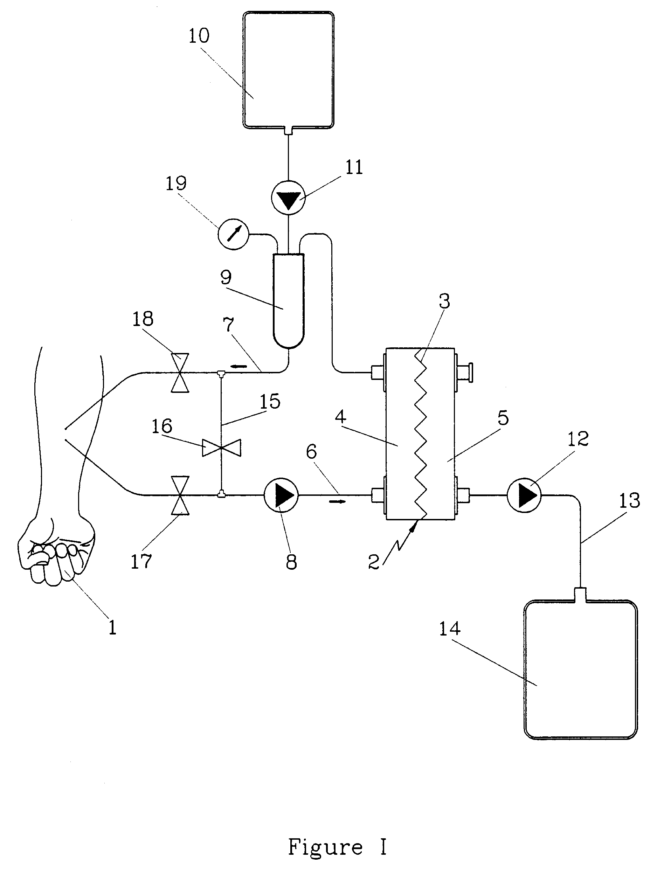

Selon le mode représenté sur la figure 1, le sang à traiter provenant d'un patient 1 est mis en circulation extracorporelle afin d'être traité par un échangeur 2 comportant une membrane 3 apte à l'ultrafiltration du sang ; cette membrane sépare l'échangeur en deux compartiments : un premier compartiment 4 reçoit le sang à traiter alors qu'un second compartiment 5 reçoit le liquide retiré du sang par ultrafiltration. Le circuit extracorporel du sang comporte une canalisation artérielle 6 d'amenée du sang à traiter du patient 1 à l'échangeur 2, et une canalisation veineuse 7 de retour du sang traité de l'échangeur 2 au patient 1. Le sang est mis en circulation grâce à l'action d'une pompe 8 située sur la canalisation artérielle. La canalisation veineuse 7 est pourvue d'un piège à bulles 9 constituant également un site d'injection d'un liquide de substitution présent dans un réservoir 10 ; le débit d'injection du liquide est fixé par le débit de la pompe de perfusion 11.
Une fraction du sang circulant dans le premier compartiment 4 de l'échangeur est ultrafiltrée à travers la membrane 3 grâce à l'action d'une pompe 12 située sur une canalisation 13 reliant le second compartiment 5 de l'échangeur à une poche de recueil 14 de l'ultrafiltrat.

Selon l'invention, le circuit extracorporel de sang comporte également une canalisation de dérivation 15 reliant directement la canalisation artérielle 6 à la canalisation veineuse 7. Cette canalisation 15 est pourvue d'un dispositif d'obturation tel qu'un clamp 16. Elle est reliée à la canalisation artérielle en un point situé, par rapport au sens d'écoulement du sang, en amont de la pompe 8 et très proche du patient ; de même elle est reliée à la canalisation veineuse 7 en aval du piège à bulles 9 en un point très proche du patient.
La canalisation artérielle 6 (respectivement veineuse 7) est également pourvue en amont (respectivement en aval) du point d'intersection avec la canalisation de dérivation 15 d'un dispositif d'obturation tel qu'un clamp 17 (respectivement 18).

Selon une caractéristique de l'invention, le piège à bulles 9 est muni d'un capteur 19 apte à indiquer la pression existant dans le circuit extracorporel.

Le fonctionnement du rein artificiel ainsi décrit est le suivant :
Lors du fonctionnement classique en mode hémofiltration, le clamp 16 est fermé et les clamps 17 et 18 sont ouverts ; la pompe 8 est commandée au débit souhaité pour la circulation du sang à l'intérieur du circuit extracorporel; la mise en oeuvre de la pompe d'extraction 12 est commandée pour provoquer une dépression dans le compartiment 5 de l'échangeur et par conséquent une ultrafiltration du sang à travers la membrane 3 ; le débit d'ultrafiltration du sang correspond au débit de la pompe 12. Après son passage dans l'échangeur, le sang est restitué au patient par la canalisation veineuse 7. Afin de compenser en totalité ou en partie la perte de liquide provoquée par l'ultrafiltration, un liquide de substitution est ajouté au sang avant son retour au patient. Cet ajout est réalisé par injection dans le piège à bulles 9 d'un liquide provenant du réservoir 10. Le débit d'injection du liquide est fixé par le débit de la pompe d'injection 11. Lorsqu'il n'est pas nécessaire de provoquer une perte de poids du patient, on commande le débit de la pompe d'injection 11 pour qu'il soit égal au débit de la pompe d'extraction 12. Ainsi, tout le volume de liquide extrait du sang par ultrafiltration est compensé par injection d'un volume identique de liquide de substitution.

Selon l'invention, la vérification de cette exacte compensation est réalisée de la façon suivante. On ouvre le clamp 16 et on ferme simultanément les clamps 17 et 18. Le patient est ainsi isolé du circuit extracorporel. Le fonctionnement des pompes 8, 11 et 12 est maintenu. Le sang recircule alors à l'intérieur d'une boucle fermée constituée par la canalisation artérielle 6, le compartiment 4 de l'échangeur, la canalisation veineuse 7 et la canalisation de dérivation 15. L'évolution de la pression à l'intérieur du circuit extracorporel peut être suivie grâce aux indications fournies par le capteur 19, relié éventuellement à un dispositif d'enregistrement graphique (non représenté), ou à un dispositif de traitement du signal. Dans le cas où le débit d'extraction de la pompe 12 est exactement égal au débit d'injection de la pompe 11, la valeur de la pression indiquée par le capteur 19 reste constante ou du moins sensiblement constante. En effet, il est possible de noter des variations de pression dues aux à-coups des pompes, la valeur moyenne de pression étant maintenue constante. Par contre, lorsque les débits des pompes étant supposés égaux, on note cependant une variation de pression, cela signifie que le débit réel fourni par l'une au moins des pompes, n'est pas égal au débit de consigne. Ainsi, si la pression indiquée par le capteur 19 augmente, cela veut dire que le débit d'injection est supérieur au débit d'extraction. Si au contraire, la pression diminue, cela veut dire que le débit d'injection est inférieur au débit d'extraction. Il est alors possible de corriger l'une ou l'autre des pompes pour obtenir une compensation réelle de la quantité de liquide ultrafiltré par une égale quantité de liquide substitué.
En outre, on peut selon l'invention effectuer un étalonnage de l'une des pompes par rapport à l'autre. En effet, il est possible de repérer la valeur de chacun des débits de consigne ou affichés des pompes 11 et 12 qui permet d'obtenir une stabilité de la pression et donc une compensation réelle. Par exemple, il se peut que, à cause des erreurs intrinsèques à chacune des pompes, l'ultrafiltration provoquée par la mise en oeuvre de la pompe d'extraction 12 à un débit affiché de 100 ml/min ne soit réellement compensée que par une injection de liquide au moyen de la pompe d'injection 11 ayant un débit affiché de 102 ml/min et non pas de 100 ml/min.

Ce rapport des débits affichés, ou de consigne , peut être maintenu en mémoire (non représentée) et être utilisé ultérieurement pour corriger la valeur de consigne des débits si l'on veut une réelle compensation du liquide ultrafiltré par le liquide de substitution.

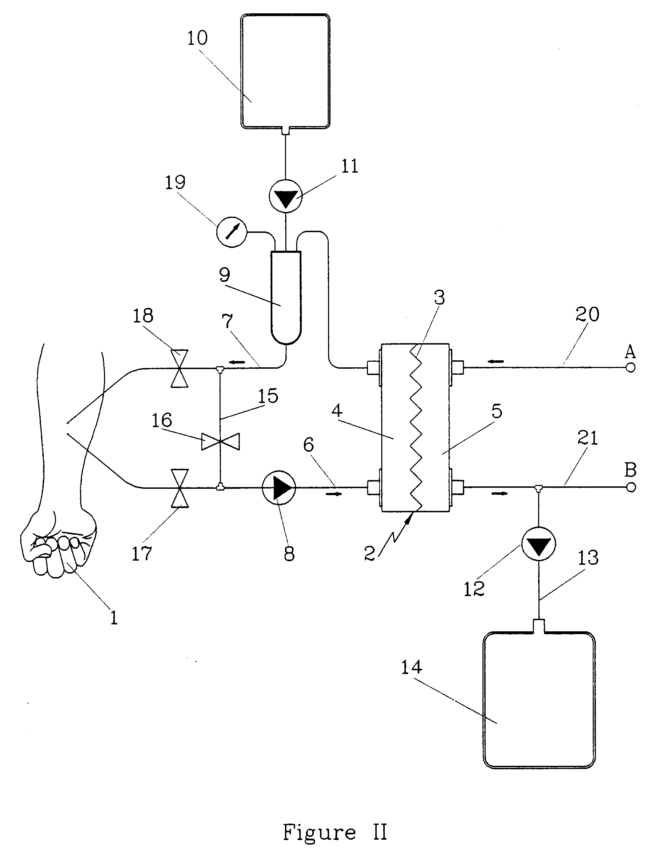

Selon le mode de réalisation de la figure 2, sur laquelle les éléments identiques à ceux de la figure 1 sont indiqués par les mêmes numéros de référence, le compartiment 5 de l'échangeur 2 est parcouru par du liquide de dialyse. En effet, une canalisation 20 conduit le liquide de dialyse à partir d'une source (non représentée) vers le compartiment 5, alors qu'une canalisation 21 conduit le liquide de dialyse du compartiment 5 vers des moyens d'évacuation ou de régénération. Le liquide s'écoulant dans la canalisation 21 s'est chargé en impuretés provenant du sang et qui, par l'effet de diffusion dû à une différence de concentration de part et d'autre de la membrane, sont passés à travers la membrane. En outre, le liquide de dialyse est, en sortie du compartiment 5, augmenté d'un volume de liquide ultrafiltré du sang par l'effet de convection ; cet effet de convection est dû à une différence de pression de part et d'autre de la membrane. Ainsi, selon la représentation de la figure 2, si le débit de liquide au point A de la canalisation 20 de liquide de dialyse est maintenu égal au débit de liquide au point B de la canalisation 21, l'ultrafiltration est provoquée par la dépression créée par la pompe d'extraction 12 située sur la canalisation 13 dont le débit est égal au débit d'ultrafiltration. De la même façon que dans le mode de réalisation précédemment décrit, la quantité de liquide soustraite du sang par ultrafiltration est compensée par une égale quantité de liquide de substitution injectée grâce à la pompe d'injection 11 dont le débit de consigne est le même que celui de la pompe d'extraction 12. La technique mise en oeuvre ici pour l'épuration du sang est l'hémodiafiltration.

Selon l'invention, pour vérifier l'exactitude de la compensation réelle entre le liquide ultrafiltré et le liquide injecté, on procède ainsi que cela a été décrit précédemment. On ouvre le clamp 16 en fermant simultanément les clamps 17 et 18 afin d'isoler le patient 1 et créer ainsi une boucle de recirculation du sang à l'intérieur du circuit extracorporel. L'indication, grâce au capteur 19, du maintien de la pression à une valeur sensiblement constante est la preuve d'une compensation parfaite. On peut alors, de même que dans le mode de réalisation précédent procéder à l'étalonnage relatif de l'une des pompes par rapport à l'autre.

Selon le mode décrit ici, le circuit de liquide de dialyse est un circuit ouvert, avec une canalisation 20 d'amenée du liquide de dialyse et une canalisation 21 de retour du liquide de dialyse ; la présente invention trouve également une application dans le cas où le circuit de liquide de dialyse possède une portion fermée, incluant le compartiment 5 de l'échangeur et sur laquelle vient se connecter la canalisation 13 d'extraction d'une quantité de liquide égale à la quantité de liquide extraite du sang par ultrafiltration.

La mise en oeuvre du cycle de vérification de la compensation exacte entre le débit de liquide ultrafiltré et le débit de liquide de substitution injecté peut se faire de façon automatique, selon une fréquence prédéterminée, pendant le traitement du sang. Dans ce cas, la commande de l'ouverture et de la fermeture des vannes peut être effectuée par une unité de contrôle ; de même les informations transmises par le capteur de pression 19 peuvent être enregistrées et traitées par cette unité de contrôle. Il est bien évident que la fréquence et la durée du cycle de vérification doivent être choisies pour ne pas nuire à l'efficacité de l'ensemble de la séance de traitement du sang. Mais grâce à la présente invention , il est possible de détecter très rapidement une déviation de l'équilibre entre la quantité de liquide extraite du sang et la quantité de liquide réinjectée au sang. En effet, lors d'essais réalisés avec un débit d'ultrafiltration et d'injection de 1,8 l/h, il a été possible d'obtenir, en 1 minute, une appréciation visuelle sur un enregistrement graphique d'une déviation de 50 ml/h.

La présente invention a été décrite pour des cas où les débits de consigne des pompes d'extraction 12 et d'injection 11 étaient égaux.
Dans le cas où on souhaite obtenir une perte de poids du patient, et où donc on fixe un débit d'extraction supérieur au débit d'injection, il est possible de déterminer, à partir des caractéristiques du circuit et de l'échangeur 2, l'évolution souhaitée de la pression à l'intérieur de la boucle de recirculation pour une certaine différence de débit ; la vérification de l'exactitude des débits fournis est alors réalisée par l'observation de l'évolution réelle de la pression puis la comparaison avec l'évolution souhaitée.
De façon alternative, même si le traitement du sang est réalisé dans des conditions de débit différentes pour la pompe d'extraction et la pompe d'injection, il est possible lors de la mise en oeuvre du procédé de vérification selon l'invention, de réaliser temporairement l'égalité des débits, d'étalonner les 2 pompes relativement l'une par rapport à l'autre, et de poursuivre la séance de traitement en tenant compte pour les débits de consigne des données obtenues pendant l'étalonnage.

L'objet de la présente invention a notamment pour avantage d'utiliser, pour le contrôle de la balance des fluides, une grandeur physique ( la pression) différente par nature de celles que l'on souhaite vérifier (débit, poids). Cet avantage peut conduire à intégrer ce procédé de contrôle dans le système de sécurité du dispositif de traitement.

La présente invention trouve également une application, en dehors des séances de traitement du sang, lors des opérations de maintenance et de vérification des appareils de dialyse. Dans ce cas, le liquide circulant dans le circuit extracorporel de sang n'est pas du sang, mais l'étalonnage relatif de la pompe d'extraction par rapport à la pompe d'injection peut être réalisé de façon simple et sûre.

La présente invention trouve également une application dans les opérations de traitement du sang par plasmaphérèse où le plasma extrait du sang est remplacé par un liquide de substitution.

## Revendications

1. Appareil de traitement du sang comprenant :
- un échangeur à membrane (2) ayant un premier et un second compartiments (4, 5) séparés par une membrane (3);
- un circuit pour circulation extracorporelle de sang, comprenant:
• une première canalisation (6) connectée à une entrée du premier compartiment (4) et destinée à être connectée à un patient (1);
• une seconde canalisation (7) connectée à une sortie du premier compartiment (4) et destinée à être connectée au patient (1);
• une canalisation de dérivation (15) reliant la première canalisation (6) à la seconde canalisation (7) de façon à définir une boucle de recirculation;
- des moyens d'obturation (16) pour obturer la canalisation de dérivation (15) ;
- des moyens d'injection (10, 11) pour injecter un liquide dans le circuit pour circulation extracorporelle de sang ;
- des moyens d'extraction (12) pour provoquer l'ultrafiltration de liquide plasmatique dans le second compartiment au travers de la membrane (3) ;
caractérisé en ce que :
- les moyens d'injection (10, 11) sont reliés à la boucle de recirculation pour y injecter un liquide de substitution destiné à compenser au moins en partie le liquide ultrafiltré,
et en ce qu'il comporte:
- des moyens d'obturation (17, 18) pour obturer la première canalisation (6) et la seconde canalisation (7) de façon à isoler le patient (1) de la boucle de recirculation ;
- des moyens de mesure de pression (19) connectés à la boucle de recirculation pour produire un signal indicatif de la différence entre un débit du liquide infusé et un débit de liquide ultrafiltré lorsque la boucle de recirculation est isolée du patient (1).

2. Appareil selon la revendication 1, comprenant en outre des moyens pour réguler au moins l'un des moyens d'injection (10, 11) et des moyens d'extraction (12) à partir du signal produit par les moyens de mesure de pression (19).

3. Appareil selon la revendication 2, caractérisé en ce que les moyens de régulation comprennent une unité de commande connectée aux moyens de mesure de pression (19), aux moyens d'injection (10, 11) et aux moyens d'extraction (12).

4. Appareil selon une des revendications 1 à 3, caractérisé en ce qu'il comporte en outre des moyens pour étalonner les moyens d'injection (10, 11) [respectivement les moyens d'extraction (12)] par rapport aux moyens d'extraction (12) [respectivement aux moyens d'injection (12)] à partir du signal produit par des moyens de mesure de pression.

5. Appareil selon la revendication 4, caractérisé en ce que les moyens d'étalonnage comprennent une unité de commande connectée aux moyens de mesure de pression (19), aux moyens d'injection (10, 11) et aux moyens d'extraction (12).

6. Appareil selon une des revendications 1 à 5, caractérisé en ce que les moyens d'injection comprennent une pompe à débit variable (11).

7. Appareil selon une des revendications 1 à 6, caractérisé en ce que les moyens d'extraction comprennent une pompe à débit variable (12).

8. Appareil selon une des revendications 1 à 7, caractérisé en ce qu'il comprend en outre une pompe (8) pour la circulation du sang disposée sur la première canalisation (6) à l'intérieur de la boucle de recirculation.

9. Procédé pour régler l'injection d'un liquide et l'extraction d'un liquide dans et hors d'un circuit extracorporel de sang connecté à un patient (1), ce procédé comprenant des étapes de:
- isoler temporairement une boucle de recirculation par rapport au patient (1), la boucle comprenant une portion d'une première canalisation (6) reliant le patient (1) à un échangeur à membrane (2), l'échangeur à membrane (2), une portion d'une seconde canalisation (7) reliant l'échangeur à membrane (2) au patient (1), et une canalisation de dérivation (15) connectant la première canalisation (6) à la seconde canalisation (7);
- faire circuler le sang dans la boucle;
- mesurer la pression dans la boucle pendant un intervalle de temps, et, lorsque la pression mesurée varie pendant l'intervalle de temps,
- modifier soit le débit d'injection de liquide dans la boucle, soit le débit d'extraction de liquide hors de la boucle de façon que la pression dans la boucle soit sensiblement constante, ou évolue selon une évolution souhaitée.

10. Procédé selon la revendication 9, comportant en outre l'étape d'établir une corrélation entre un débit d'extraction apparent et un débit d'injection apparent.

11. Procédé selon la revendication 10, caractérisé en ce qu'il est appliqué à l'hémofiltration du sang.

## Claims

1. A blood treatment apparatus comprising:
- a membrane exchanger (2) having a first and a second compartments (4, 5) separated by a membrane (3);
- an extracorporeal blood circuit comprising :
• a first tubing (6) connected to an inlet of the first compartment (4) and intended to be connected to a patient (1);
• a second tubing (7) connected to an outlet of the first compartment (4) and designed to be connected to the patient (1);
• a bypass line (15) linking the first tubing (6) to the second tubing (7) in order to define a recirculation loop;
- obturating means (16) to obturate the bypass line (15);
- injection means (10, 11) to inject a liquid in the extracorporeal blood circuit;
- withdrawal means (12) to bring about the ultrafiltration of plasmatic liquid in the second compartment through the membrane (3);
characterized in that:
- the injection means (10, 11) are linked to the recirculation loop in order to inject therein a liquid of substitution intended to compensate at least partly for the ultrafiltrated liquid
and in that it comprises:
- obturating means (17, 18) to obturate the first tubing (6) and the second tubing (7) in order to isolate the patient (1) from the recirculation loop;
- pressure measuring means (19) connected to the recirculation loop for generating a signal indicative of the difference between a flowrate of the infused liquid and a flowrate of the ultrafiltrated liquid when the recirculation loop is isolated from the patient (1).

2. Device according to claim 1, comprising furthermore means to regulate at least one of the injection means (10, 11) and withdrawal means (12) from the signal generated by the pressure measuring means (19).

3. Device according to claim 2, characterized in that the regulating means comprise a control unit connected to the pressure measuring means (19), to the injection means (10, 11) and to the withdrawal means (12).

4. Device according to claims 1 to 3, characterized in that it further comprises means to calibrate the injection means (10, 11) [respectively to the withdrawal means (12)] in relation to the withdrawal means (12) [respectively to the injection means (10, 11)] from the signal generated by the pressure measuring means.

5. Device according to claim 4, characterized in that the calibrating means comprise a control unit connected to the pressure measuring means (19), to the injection means (10, 11) and to the withdrawal means (12).

6. Device according to one of claims 1 to 5, characterized in that the injection means comprise a variable flowrate pump (11).

7. Device according to one of claims 1 to 6, characterized in that the withdrawal means comprise a variable flowrate pump (12).

8. Device according to one of claims 1 to 7, characterized in that it further comprises a pump (8) for blood circulation located on the first tubing (8) inside the recirculation loop.

9. Method for controlling liquid injection and withdrawal inside and outside an extracorporeal blood circuit connected to a patient (1), this method comprising the steps of:
- temporarily isolating a recirculation loop in relation to the patient (1), the loop comprising a part of a first tubing (6) linking the patient (1) to a membrane exchanger (2), the membrane exchanger (2), a part of a second tubing (7) linking the membrane exchanger (2) to the patient (1), and a by-pass line (15) connecting the first tubing (6) to the second tubing (7);
- circulating blood through the loop;
- measuring the pressure in the loop over a period of time, and, when the measured pressure varies over the period of time;
- modifying either the injection flowrate of liquid into the loop, or the withdrawal flowrate of liquid from the loop in order that the pressure is substantially constant, or changes according to a desired evolution.

10. Method according to claim 9, further comprising the step of establishing a correlation between an apparent withdrawal flowrate and an apparent injection flowrate.

11. Method according to claim 10, characterized in that it applies to blood heamofiltration.

## Patentansprüche

1. Blutbehandlungsgerät, das folgendes umfaßt:
- einen Membranaustauscher (2) mit einem durch eine Membran (3) getrennten ersten und zweiten Abteil (4, 5);
- einen Kreislauf zum extrakorporalen Zirkulieren von Blut mit:
• einer ersten mit einem Eingang des ersten Abteils (4) verbundenen Leitung (6), die an einen Patienten (1) angeschlossen werden soll;
• einer zweiten mit einem Ausgang des ersten Abteils (4) verbundenen Leitung (7), die an den Patienten (1) angeschlossen werden soll;
• einer Abzweigleitung (15), die die erste Leitung (6) mit der zweiten Leitung (7) verbindet so daß eine Rezirkulationsschleife gebildet ist;
- Sperrmittel (16) zum Sperren der Abzweigleitung (15);
- Injektionsmittel (10, 11) zum Injizieren einer Flüssigkeit in den Kreislauf zum extrakorporalen Zirkulieren von Blut;
- Abziehmittel (12) zum Bewirken der Ultrafiltration von Plasmaflüssigkeit im zweiten Abteil durch die Membran (3) hindurch,
dadurch gekennzeichnet, daß
- die Injektionsmittel (10, 11) mit der Rezirkulationsschleife verbunden sind, um dort eine Substitutionsflüssigkeit zu injizieren, die die ultrafiltrierte Flüssigkeit mindestens teilweise kompensieren soll,
und daß es folgendes umfaßt:
- Sperrmittel (17, 18), um die erste Leitung (6) und die zweite Leitung (7) so zu sperren, daß der Patient (1) von der Rezirkulationsschleife abgeschlossen wird,
- Druckmeßmittel (19), die an die Rezirkulationsschleife angeschlossen sind und ein Signal erzeugen, das die Differenz zwischen einer Menge an injizierter Flüssigkeit und einer Menge an ultrafiltrierter Flüssigkeit angibt, wenn die Rezirkulationsschleife vom Patienten (1) abgeschlossen wird.

2. Gerät nach Anspruch 1, das weiterhin Mittel zum Regulieren mindestens eines der Injektionsmittel (10, 11) und der Abziehmittel (12) auf der Grundlage des von den Druckmeßmitteln (19) erzeugten Signals umfaßt.

3. Gerät nach Anspruch 2, dadurch gekennzeichnet, daß die Regulierungsmittel eine mit den Druckmeßmitteln (19), den Injektionsmitteln (10, 11) und den Abziehmitteln (12) verbundene Steuereinheit umfassen.

4. Gerät nach einem der Ansprüche 1 bis 3, dadurch gekennzeichnet, daß es des weiteren Mittel zum Eichen der Injektionsmittel (10, 11) [beziehungsweise der Abziehmittel (12)] bezüglich der Abziehmittel (12) [beziehungsweise der Injektionsmittel (10, 11)] auf der Grundlage des von den Druckmeßmitteln erzeugten Signals umfaßt.

5. Gerät nach Anspruch 4, dadurch gekennzeichnet, daß die Eichmittel eine mit den Druckmeßmitteln (19), den Injektionsmitteln (10, 11) und den Abziehmitteln (12) verbundene Steuereinheit umfassen.

6. Gerät nach einem der Ansprüche 1 bis 5, dadurch gekennzeichnet, daß die Injektionsmittel eine Regelpumpe (11) umfassen.

7. Gerät nach einem der Ansprüche 1 bis 6, dadurch gekennzeichnet, daß die Abziehmittel eine Regelpumpe (12) umfassen.

8. Gerät nach einem der Ansprüche 1 bis 7, dadurch gekennzeichnet, daß es weiterhin eine an der ersten Leitung (6) im Innern der Rezirkulationsschleife angeordnete Pumpe (8) für das Zirkulieren des Blutes umfaßt.

9. Verfahren zur Regelung der Injektion einer Flüssigkeit in einen und des Abziehens einer Flüssigkeit aus einem an einen Patienten (1) angeschlossenen extrakorporalen Blutkreislauf, wobei dieses Verfahren die folgenden Schritte umfaßt:
- eine Rezirkulationsschleife wird vorübergehend von einem Patienten (1) abgeschlossen, wobei die Schleife einen Teil einer ersten Leitung (6), die den Patienten (1) mit einem Membranaustauscher (2) verbindet, den Membranaustauscher (2), einen Teil einer zweiten Leitung (7), die den Membranaustauscher (2) mit dem Patienten (1) verbindet, und eine Abzweigleitung (15), die die erste Leitung (6) mit der zweiten Leitung (7) verbindet, umfaßt;
- das Blut wird in der Schleife zirkuliert;
- der Druck in der Schleife während eines Zeitraums wird gemessen, und wenn der gemessene Druck während des Zeitraums schwankt,
- wird entweder die Menge an in die Schleife injizierte Flüssigkeit oder die Menge an aus der Schleife abgezogene Flüssigkeit so modifiziert, daß der in der Schleife herrschende Druck im wesentlichen konstant ist oder sich wie gewünscht entwickelt.

10. Verfahren nach Anspruch 9, bei dem man außerdem die scheinbare Abzugsmenge mit der scheinbaren Injektionsmenge korreliert.

11. Verfahren nach Anspruch 10, dadurch gekennzeichnet, daß es bei der Hämofiltration des Blutes Anwendung findet.
